# EUROPEAN PATENT SPECIFICATION

(11) **EP 3 284 340 B1**
(45) Date of publication and mention of the grant of the patent: **07.10.2020**
(21) Application number: 17184866.6
(22) Date of filing: 04.08.2017
(51) Int. Cl.: A01K 29/00, A01K 45/00, A61N 5/00

(54) **REARING METHOD FOR GROWING CATTLE**
AUFZUCHTVERFAHREN ZUR ZÜCHTUNG VON VIEH
PROCÉDÉ D'ÉLEVAGE DE BÉTAIL

(30) Priority: 19.08.2016 JP 2016161133
(43) Date of publication of application: 21.02.2018
(73) Proprietor: Showa Denko K.K., Tokyo 105-8518 (JP); Hiroshima University, Higashi-Hiroshima-shi Hiroshima 739-8511 (JP)
(72) Inventor: Shinoda, Akiko, Tokyo, 105-8518 (JP); Watanabe, Takashi, Tokyo, 105-8518 (JP); Sugino, Toshihisa, Hiroshima, 739-8528 (JP); Mon, Mamiko, Hiroshima, 739-8528 (JP)
(74) Representative: Strehl Schübel-Hopf & Partner

(56) References cited:
- JP-A- 2009 519 021
- US-A1- 2005 072 367
- H House: "Bringing the Resources of the World to Rural Ontario Energy Opportunities LIGHTING FOR MORE MILK", , 1 January 2006 (2006-01-01), XP055631701, Retrieved from the Internet: URL:http://www.omafra.gov.on.ca/english/en gineer/facts/06-053.pdf
- JOU JWO-HUEI ET AL: "A universal, easy-to-apply light-quality index based on natural light spectrum resemblance", APPLIED PHYSICS LETTERS, A I P PUBLISHING LLC, US, vol. 104, no. 20, 19 May 2014 (2014-05-19) , XP012185820, ISSN: 0003-6951, DOI: 10.1063/1.4879635 [retrieved on 1901-01-01]
- STEPHEN HUGHES ET AL: "A candle in the lab", PHYSICS EDUCATION, INSTITUTE OF PHYSICS PUBLISHING, BRISTOL, GB, vol. 42, no. 3, 1 May 2007 (2007-05-01), pages 271-274, XP020112830, ISSN: 0031-9120, DOI: 10.1088/0031-9120/42/3/006

## Description

### BACKGROUND OF THE INVENTION

### 1. Field of the Invention

The present disclosure relates to a rearing method for growing cattle.

### 2. Description of the Related Art

It is important for high daily gain of body weight by increase food intake via stimulate the appetite in growing cattle management. Therefore, feed mixtures have been proposed that raise the energy intake of growing cattle, and increase the productivity (see, for example, Patent document 1).

Meanwhile, melatonin has been known as a hormone that adjusts a circadian rhythm of an animal. Melatonin is a substance secreted in response to the cycle of day and night, which exhibits a rhythmic pattern of being high in the night and low during the daytime. Note here that when melatonin is secreted, body fluid decreases, and thereby, the milk production also decreases. Therefore, long-day management has become popular by which dairy cattle in the lactating period is managed in a lighting environment where the light period is longer than the dark period, in order to increase the milk production.

In addition, it has been known that secretion of melatonin is strongly influenced by light during the daytime, especially, short-wavelength light (blue light) (see, for example, Patent document 2 and Non-patent document 1).

### Related-Art Documents

### Patent Documents

[Patent Document 1] Japanese Unexamined Patent Application Publication No. 2008-5809
[Patent Document 2] Japanese Translation of PCT International Application Publication No. 2009-519021

### Non-patent documents

[Non-patent document 1] Chronobiology Vol.14, No.1, P.13-20 (2008).

However, it has been desired to enhance the appetite in growing cattle, without greatly disturbing the circadian rhythm.

In view of the above, it is an object of the present disclosure to provide a rearing method for growing cattle by which the appetite for feed can be enhanced, without greatly disturbing the circadian rhythm.

### SUMMARY OF THE INVENTION

There is a report on deer and sheep that, compared to the winter season, there are increases in food intake, in the metabolism efficiency, and also in the body weight in the summer season. On the other hand, there is a report on dairy cattle that no seasonal variation of the body weight is observed despite also being ruminants.

The inventors have investigated variations of methods of long-day management in order to expedite the growth of cattle in a growing process, and as a result, completed the invention with the finding that growing cattle irradiated with light under a predetermined condition shows a high appetite for feed under the long-day management, without greatly disturbing the circadian rhythm of the growing cattle.

In other words, the present disclosure provides the following method in order to solve the above problem.
(1) The feed management for growing cattle, based on a circadian cycle, under a long-day management that manages the growing cattle in a lighting environment in which a light period is longer than a dark period, includes having the light period include a first period during which the growing cattle are irradiated with light including blue light, and a second period following the first period, during which the growing cattle are irradiated with light not including blue light, but including amber light.
(2) The rearing method for growing cattle according to (1), wherein the circadian cycle is within a range of 23 to 25 hours, the light period is within a range of 15 to 17 hours, and the dark period is within a range of 9 to 7 hours.
(3) The rearing method for growing cattle according to (2), wherein the second period is within a range of 1 to 3 hours.
(4) The rearing method for growing cattle according to any one of (1) to (3), wherein the light not including blue light but including amber light includes light having a peak wavelength within a range of 570 nm to 610 nm, and does not include light whose wavelength is within a range of 380 nm to 500 nm.

According to the invention, it is possible to provide a rearing method for growing cattle by which the appetite for feed can be enhanced, without greatly disturbing the circadian rhythm.

### BRIEF DESCRIPTION OF DRAWINGS

FIG. 1 is a diagram illustrating a rearing method for growing cattle according to an embodiment in the present disclosure;
FIG. 2 is an emission spectrum of white LED lighting used in a lighting environment during a first period in an application example 1;
FIG. 3 is an emission spectrum of white LED lighting used in a lighting environment during a second period in the application example 1;
FIG. 4 is a diagram illustrating a change in plasma melatonin concentration in Holstein calves in the application example 1 and a comparative example 1; and
FIG. 5 is a diagram illustrating a change in plasma GLP-1 concentration in Holstein calves in the application example 1 and the comparative example 1.

### DETAILED DESCRIPTION OF THE EMBODIMENTS

When investigating a rearing method for growing cattle by which the appetite for feed can be enhanced, without greatly disturbing the circadian rhythm, the inventors have paid attention to plasma glucagon like peptide-1 (GLP-1) concentration in growing cattle.

GLP-1 is a hormone secreted from the small intestine after feeding, and has a function to stimulate insulin secretion, and at the same time, to suppress the appetite. In other words, a high GLP-1 concentration of the blood plasma suppresses the appetite.

According to the research by the inventors, although no difference has been found in the secreted volume of GLP-1 during the daytime between long-day management and short-day management used for growing cattle, a result was obtained that the secreted volume of GLP-1 in the night was greater under the short-day management than in the long-day management. This result showed that an amount of feed intake of the growing cattle under the short-day management being smaller than that under the long-day management is controlled by secretion of GLP-1.

In other words, a low GLP-1 level was secreted under the long-day management compared with the short-day management, and thereby, the feed intake became greater. In other words, blue light during the daytime to which the cattle were exposed seemed to influence the secretion of GLP-1. Additionally, there is a report on irradiation of blue light used for medical treatment of anorexia, which may imply a high possibility that blue light takes part in suppressing secretion of GLP-1.

Meanwhile, according to examination of the inventors, if amber light or blue light is used for growing cattle under the long-day management at the end of the light period, although no difference was observed between both cases in terms of the secreted volume of GLP-1 during the end of the light period and just before the end, using the blue light increased the secreted volume of GLP-1 during the dark period compared to the case of using the amber light. At the same time, a result was obtained that using the amber light increased intake of hay, compared to the case of using the blue light.

According to examination of the inventors, contrary to a conventional common sense that blue light causes secretion suppression of GLP-1, long-time continuous exposure to blue light under the long-day management disturbs secretion of GLP-1, and meanwhile, introducing amber light at the end of the long-day management lowered the secreted volume of GLP-1, and consequently, increased the intake of hay.

As described above, for the rearing of growing cattle, the inventors have found a new method of introducing amber light for several hours at the end of the long-day management, by which the intake of hay can be increased, and growth can be expedited. Furthermore, an increased intake of hay can also contribute to development of the upper gastrointestinal tract (rumen) while growing, and consequently, lead to better fattening of beef cattle, and milk performance improvement of dairy cattle.

A rearing method for growing cattle according to the embodiment is a method of rearing the growing cattle based on a circadian cycle, under long-day management in which the cattle are managed in a lighting environment where the light period is longer than the dark period; and the light period includes a first period during which the growing cattle are irradiated with light including blue light, and a second period following the first period, during which the growing cattle are irradiated with light not including blue light, but including amber light.

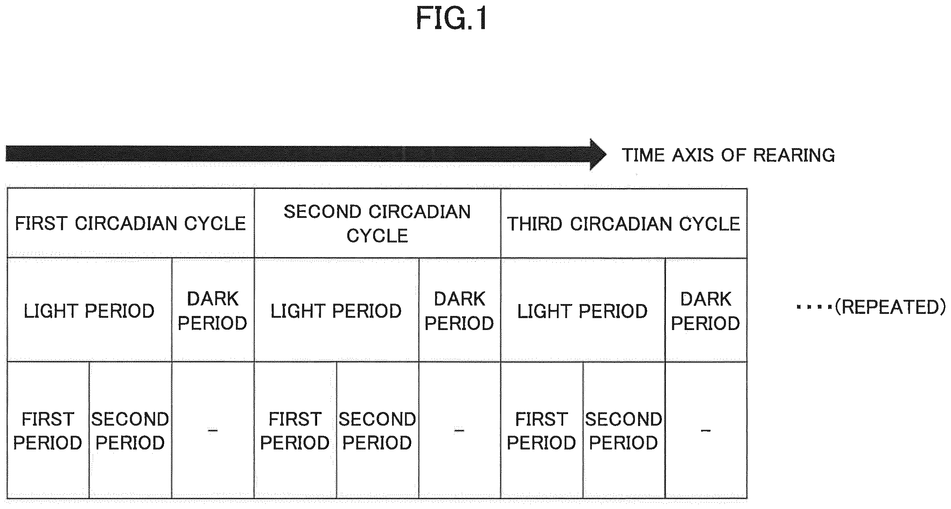

Next, a rearing method for growing cattle will be described according to the embodiment, using FIG. 1.

The rearing method for growing cattle according to the embodiment is executed based on a circadian cycle. Here, "circadian cycle" is an endogenous rhythm seen in physiological phenomena of an animal such as activity and sleep. The circadian cycle is a cycle of about 24 hours in general, but the circadian cycle fluctuates depending on stimulus from the outside world, such as light, temperature, and feeding. In other words, although the circadian cycle in the embodiment is a cycle of about 24 hours, it may fluctuate within a time range of about one hour, depending on the type of cattle and the rearing environment. That is, the circadian cycle fluctuates depending on the type of cattle and the rearing environment, and falls within a range between about 23 and 25 hours.

The rearing method for growing cattle according to the embodiment is executed by repeating a circadian cycle of about 24 hours as illustrated in FIG. 1. Adopting such a method enables a smaller disturbance of the circadian rhythm for the growing cattle, and it is possible to avoid raising stress of the growing cattle, and to avoid reduced efficiency of breeding or growing.

The rearing method for growing cattle according to the embodiment is executed under long-day management. The "long-day management" refers to managing in a lighting environment in which the length of the daytime is longer than the night (in the embodiment, the "daytime" may be referred to as the "light period", and the "night" may be referred to as the "dark period") . In other words, lengths of the daytime and the night change following the seasons; for example, in the Northern Hemisphere, the length of the daytime is longest in the summer solstice, and is shortest in the winter solstice. Even in short-daytime days in such a seasonal variation, the rearing method for cattle according to the embodiment provides a long-day lighting environment by using supplemental lightening. Since cattle act vigorously especially in the light period, adopting such a rearing method makes it possible to enhance the appetite for feed, and to expedite the growth of the growing cattle.

As illustrated in FIG. 1, the rearing method for growing cattle according to the embodiment is based on the long-day management constituted with a light period and a dark period, in which a light period consists of a first period and a second period following the first period. Here, during the first period, the growing cattle are irradiated with light including blue light, and during the second period, the growing cattle are irradiated with light not including blue light but including amber light.

The light period in the embodiment is preferably set within a range of 15 to 17 hours, and is more preferably set within a range of 15.75 to 16.25 hours. Setting the light period greater than or equal to 15 hours and less than or equal to 17 hours can increase the calorie intake of the growing cattle, and can raise the productivity.

The first period according to the embodiment is a period during which the growing cattle are irradiated with light including blue light, and is more preferably a period during which the growing cattle are irradiated with light including light having a wavelength within a range of 380 nm to 500 nm. The lighting environment of the first period may be provided, for example, by introducing the sunlight into a cattle shed, and turning on a device emitting white light disposed in the cattle shed. In the embodiment, fluorescent light, white LED lighting, incandescent light (tungsten bulbs), and the like may be used as the device emitting white light. However, if using incandescent light (tungsten bulbs), the illuminance needs to be set greater than or equal to a predetermined value, for light having wavelengths within a range of 380 nm to 500 nm.

Specific effects of the lighting environment during the first period include waking up the growing cattle, stimulating the growing cattle to act vigorously, and resetting the biological clocks; and thereby raising the appetite of the growing cattle, and consequently, improving the efficiency of the growing, and stabilizing the circadian rhythm of the growing cattle.

Also, the first period in the light period is preferably within a range of 13 to 15 hours, and is more preferably within a range of 13.75 to 14.25 hours. Setting the first period greater than or equal to 13 hours and less than or equal to 15 hours can further stimulate the growing cattle to act vigorously, and can enhance the appetite for feed.

As illustrated in FIG. 1, the second period according to the embodiment indicates a period before sunset at which the light period becomes the dark period, during which the growing cattle are irradiated with light not including blue light but including amber light. According to examination by the inventors, by setting the lighting environment of the second period in this way, it is possible to suppress secretion of GLP-1 in the body of the growing cattle, to enhance the appetite of the growing cattle, and thereby to provide a rearing method for the growing cattle having high growth efficiency.

In the embodiment, it is preferable that the light not including blue light but including amber light includes light whose wavelengths is within a range of 570 nm to 610 nm, and does not include light whose wavelengths is within a range of 380 nm to 500 nm. By using the lighting environment of the second period as such, it is possible to suppress secretion of GLP-1 in the body of the growing cattle, to enhance the appetite of the growing cattle, and thereby to provide a rearing method for the growing cattle having high growth efficiency.

Also, the second period in the light period is preferably set within a range of 1 to 3 hours, and is more preferably set within a range of 1.75 to 2.25 hours.

The dark period according to the embodiment is preferably set within a range of 9 to 7 hours, and is more preferably set within a range of 8.25 to 7.75 hours. Setting the dark period less than or equal to 9 hours and greater than or equal to 7 hours increases the intake of hay, by which an effect can be expected in stimulating development of healthy ruminant stomachs.

The dark period is a period during which the growing cattle are not irradiated with light.

The growing cattle to which the rearing method for growing cattle according to the embodiment is applied is not specifically limited, and may include milk calves.

The breed of growing cattle to which the rearing method for growing cattle according to the embodiment is applied is not specifically limited, and may include Holsteins.

### [Embodiments]

In the following, effects of the present invention will be further clarified with application examples. The present invention is not limited to the following application examples, and may be changed appropriately within the scope of the claims.

### (Application example 1)

Six Holstein dairy calves (8 wk of age, 97±4.1 Kg BW) were reared for seven days in an insulated daylight sheds. The circadian cycle in the rearing was set to 24 hours from 5 a.m. to 5 a.m. of the following day. Here, the first period in the light period was set from 5 a.m. to 7 p.m., the second period in the light period was set from 7 p.m. to 9 p.m., and the dark period was set from 9 p.m. to 5 a.m. of the following day. These periods constitute one cycle, and this 24-hour cycle was repeated for the seven days.

The rearing environment was set to have a temperature of 22 °C and a humidity of 60%RH, and 2.5 kg/d of formula feed with klein grass hay offered ad libitum per head of the cattle.

Lighting in the cattle shed was set as follows. As a lighting environment of the first period, white LED lighting with the illuminance of 845 lx (11 µmol/(m²• s)) was used; and as a lighting environment of the second period, LED lighting with the illuminance of 46 lx (0.46 µmοl/(m²•s)) having a center wavelength of 594 nm was used. No lighting was used during the dark period.

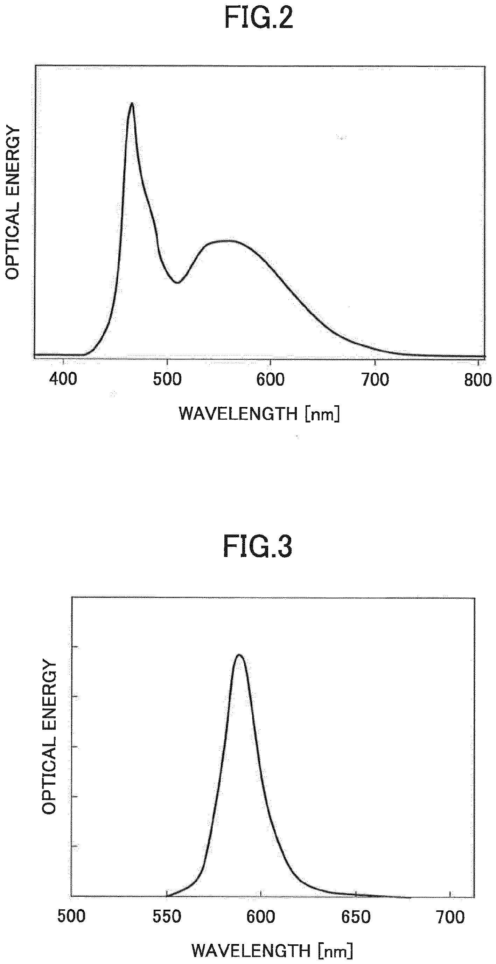

FIG. 2 and FIG. 3 illustrate the emission spectrum of the white LED lighting used for the lighting environment of the first period, and the emission spectrum of the LED lighting used for the lighting environment of the second period, respectively.

On the final day (the seventh circadian cycle) of the rearing, blood samples were taken from the six Holstein calves, and the melatonin concentration and the GLP-1 concentration (average) in the blood plasma were measured. Here, the blood samples were taken by using indwelling jugular venous catheterization.

### (Comparative example 1)

Holstein calves were reared in the same way as in the application example 1 except that the second period was not provided in the light period so that the light period was constituted only with the first period.

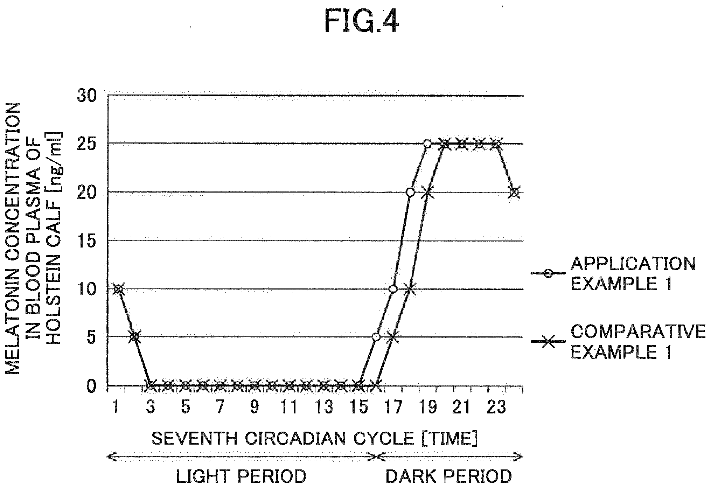

FIG. 4 illustrates a change in plasma melatonin concentration in Holstein calves in the application example 1 and the comparative example 1.

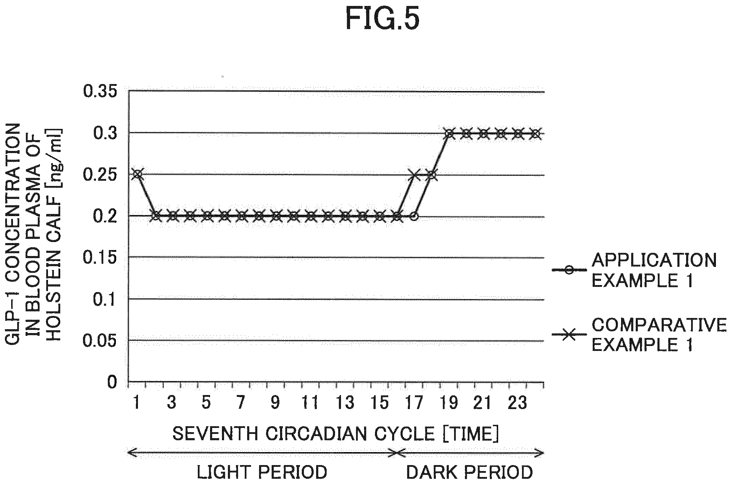

Also, FIG. 5 illustrates a change in plasma GLP-1 concentration in Holstein calves in the application example 1 and the comparative example 1.

Here, the change in plasma melatonin concentration in the Holstein calves represents the circadian rhythm of the Holstein calves.

Also, the change in plasma GLP-1 concentration in the Holstein calves represents the appetite of the Holstein calves. In other words, a lower plasma GLP-1 concentration in the Holstein calves represents a higher appetite of the Holstein calves, and a higher plasma GLP-1 concentration represents a lower appetite of the Holstein calves. Note that the GLP-1 is a hormone secreted from the small intestine after feeding, and has a function to stimulate secretion of insulin and at the same time, to suppress the appetite.

As can be seen clearly in FIG. 4, the rearing method for the Holstein calves in the application example 1 does not greatly disturb the circadian rhythm of the Holstein calves, when comparing with the rearing method for the Holstein calves in the comparative example 1.

Also, as can be seen clearly in FIG. 5, compared with the rearing method for the Holstein calves in the comparative example 1, the rearing method for the Holstein calves in the application example 1 can suppress the plasma GLP-1 concentration in the Holstein calves to a lower level at the beginning of the dark period, and thereby there is no lowering of the appetite of the Holstein, and no reduction in the appetite for feed of the Holstein calves.

## Claims

1. A rearing method for growing cattle, based on a circadian cycle, under a long-day management that manages the growing cattle in a lighting environment in which a light period is longer than a dark period, the method comprising:
having the light period include a first period during which the growing cattle are irradiated with light including blue light, and a second period following the first period, during which the growing cattle are irradiated with light not including blue light, but including amber light.

2. The rearing method for growing cattle as claimed in claim 1, wherein the circadian cycle is within a range of 23 to 25 hours, the light period is within a range of 15 to 17 hours, and the dark period is within a range of 9 to 7 hours.

3. The rearing method for growing cattle as claimed in claim 2, wherein the second period is within a range of 1 to 3 hours.

4. The rearing method for growing cattle as claimed in any one of claims 1 to 3, wherein the light not including blue light but including amber light includes light having a peak wavelength within a range of 570 nm to 610 nm, and does not include light whose wavelength is within a range of 380 nm to 500 nm.

5. The rearing method according to any one of claims 1 to 4, wherein the growing cattle are milk calves.

## Patentansprüche

1. Rinderaufzuchtverfahren, basierend auf eine Tagesrhythmus, unter einem Langzeitmanagement, das die wachsenden Rinder in einer Lichtumgebung verwaltet, in der eine Lichtperiode länger ist als eine Dunkelperiode, wobei das Verfahren umfasst:
die Lichtperiode umfasst eine erste Periode, in der die wachsenden Rinder mit Licht, einschließlich blauem Licht, bestrahlt werden, und eine zweite Periode nach der ersten Periode, in der die wachsenden Rinder mit Licht bestrahlt werden, das nicht blaues Licht, sondern gelbes Licht einschließt.

2. Rinderaufzuchtverfahren nach Anspruch 1, wobei der Tagesrhythmus innerhalb eines Bereichs von 23 bis 25 Stunden, die Lichtperiode innerhalb eines Bereichs von 15 bis 17 Stunden und die Dunkelperiode innerhalb eines Bereichs von 9 bis 7 Stunden liegt.

3. Rinderaufzuchtverfahren nach Anspruch 2, wobei der zweite Zeitraum innerhalb einer Spanne von 1 bis 3 Stunden liegt.

4. Rinderaufzuchtverfahren nach einem der Ansprüche 1 bis 3, wobei das Licht, das kein blaues Licht, sondern gelbes Licht einschließt, Licht mit einer Spitzenwellenlänge in einem Bereich von 570 nm bis 610 nm einschließt und Licht, dessen Wellenlänge in einem Bereich von 380 nm bis 500 nm liegt, nicht einschließt.

5. Rinderaufzuchtverfahren nach einem der Ansprüche 1 bis 4, bei der die wachsenden Rinder Milchkälber sind.

## Revendications

1. Procédé d'élevage de bovins en croissance, basé sur un cycle circadien, suivant une gestion de type jour long qui gère les bovins en croissance dans un environnement d'éclairage dans lequel une période de lumière est plus longue qu'une période d'obscurité, le procédé comprenant l'établissement d'une période de lumière comprenant une première période pendant laquelle les bovins en croissance sont irradiés avec une lumière qui contient une lumière bleue, et une seconde période qui suit la première période, pendant laquelle les bovins en croissance sont irradiés avec une lumière qui ne contient pas de lumière bleue mais qui contient une lumière ambrée.

2. Procédé d'élevage de bovins en croissance selon la revendication 1, dans lequel le cycle circadien est compris à l'intérieur d'une plage de 23 heures à 25 heures, la période de lumière est comprise à l'intérieur d'une plage de 15 heures à 17 heures, et la période d'obscurité est comprise à l'intérieur d'une plage de 9 heures à 7 heures.

3. Procédé d'élevage de bovins en croissance selon la revendication 2, dans lequel la seconde période est comprise à l'intérieur d'une plage de 1 heure à 3 heures.

4. Procédé d'élevage de bovins en croissance selon l'une quelconque des revendications 1 à 3, dans lequel la lumière ne contenant pas de lumière bleue mais contenant une lumière ambrée comprend une lumière qui présente une longueur d'onde de pic comprise à l'intérieur d'une plage de 570 nm à 610 nm, et ne comprend pas de lumière dont la longueur d'onde est comprise à l'intérieur d'une plage de 380 nm à 500 nm.

5. Procédé d'élevage selon l'une quelconque des revendications 1 à 4, dans lequel les bovins en croissance sont des veaux de lait.
